Europäisches Patentamt

European Patent Office

Office européen des brevets

(19)

(11) Veröffentlichungsnummer: **0 275 375 B1**

(12) # EUROPÄISCHE PATENTSCHRIFT

(45) Veröffentlichungstag der Patentschrift: 07.08.91

(21) Anmeldenummer: 87115632.9

(22) Anmeldetag: 24.10.87

(51) Int. Cl.⁵: **A61F 2/46**, A61B 17/16, B23D 71/04

(54) **Raspelartiges Räuminstrument.**

(30) Priorität: 08.01.87 CH 39/87

(43) Veröffentlichungstag der Anmeldung:
27.07.88 Patentblatt 88/30

(45) Bekanntmachung des Hinweises auf die Patenterteilung:
07.08.91 Patentblatt 91/32

(84) Benannte Vertragsstaaten:
AT DE FR GB IT

(56) Entgegenhaltungen:
DE-A- 2 621 666
DE-A- 2 732 325
FR-A- 2 284 308
FR-A- 2 547 192

(73) Patentinhaber: **GEBRÜDER SULZER AKTIENGE-
SELLSCHAFT**
**Zürcherstrasse 9**
**CH-8401 Winterthur(CH)**

(72) Erfinder: **Frey, Otto**
**Wallrütistrasse 56**
**CH-8400 Winterthur(CH)**
Erfinder: **Koch, Rudolf**
**Oberdorfstrasse 229**
**CH-8267 Berlingen(CH)**

## Beschreibung

Die Erfindung betrifft ein raspelartiges Räuminstrument zum Vorbereiten eines Röhrenknochens für den Einsatz eines Implantatschaftes, welches Instrument über seine Arbeitslänge in Umfangsrichtung scharfe Kanten hat, die beim Einschlagen in Richtung seiner Achse Knochenmaterial abschälen, wobei der Arbeitsbereich des Instrumentes als die Form des zu implantierenden Schaftes mindestens annähernd abbildender Hohlkörper ausgeführt ist, wobei ferner unter den Schneidkanten durch die Hohlkörperwand in das Innere des Hohlkörpers führende Durchbrüche vorgesehen sind.

Ein Räuminstrument der genannten Art ist aus der FR-A-2547 192 bekannt; der in Form und Abmessungen an den zu implantierenden Schaft angepasste Hohlkörper ist aussen mit sich über den ganzen Umfang erstreckenden Schneidkanten versehen, unter denen jeweils eine Reihe von über den Umfang verteilten Durchbrüchen angeordnet sind.

Bei diesem bekannten Instrument bereitet es Schwierigkeiten, den von dem Instrument abgeschälten Abraum in erforderlichen Ausmass zu entfernen; im allgemeinen "verstopft" der nicht wegzuführende Abraum daher die Schneidkanten beim Einschlagen des Instrumentes nach einer kurzen Wegstrecke. Als Folge davon wird ein Teil des spongiose Knochenmaterial beim weiteren Einschlagen nur noch verdrängt und zusammengepresst.

Aufgabe der Erfindung ist es, ein Räuminstrument der genannten Art zu schaffen, bei dem das "Austragen" des abgeschälten Abraumes vereinfacht und verbessert ist, und so die Schädigung des den zu räumenden Hohlraum umgebenden spongiosen Knochengewebes verringert oder sogar vermieden wird.

Mit der vorliegenden Erfindung wird diese Aufgabe dadurch gelöst, dass die Schneidkanten zu einzelnen, in Umfangsrichtung verteilten Zähnen aufgelöst sind, und dass ferner jedem Zahn direkt ein Durchbruch durch die Hohlkörperwand zugeordnet ist.

Beim Einschlagen des neuen Instruments dringt praktisch das ganze abgeschälte Abraummaterial durch die den Zähnen direkt zugeordneten Durchbrüche in den Innenhohlraum des Instrumentes ein, so dass die Zähne nicht mehr verstopfen können. Der grösste Teil dieses Materials wird dann beim Ausziehen des Instrumentes aus dem Hohlraum entfernt.

Die Herstellung des neuen Instrumentes wird besonders einfach, wenn der Hohlkörper aus einzelnen, längs Stosskanten mindestens stellenweise miteinander verschweissten Blechen aufgebaut ist.

Sollen Länge und Breite des Arbeitsbereiches besonders im distalen Bereich des Instrumentes variiert werden können, so ist es vorteilhaft, wenn das Instrument in Achsrichtung aus zwei Teilen zusammengesetzt ist. Die Räumarbeit kann weiterhin erleichtert werden, wenn bei einem zweiteiligen Instrument der distale Teil als zahnfreier Führungskörper ausgebildet ist. Dieser führt das Instrument in einer zuvor hergestellten zentralen Markbohrung beim Einschlagen.

Im folgenden wird die Erfindung anhand von Ausführungsbeispielen im Zusammenhang mit der Zeichnung näher erläutert.

Fig. 1    zeigt eine Ansicht des neuen Instrumentes in etwa natürlicher Grösse;

Fig. 2    und 3 sind die Schnitte A A und B B von Fig. 1;

Fig. 4    gibt in gleicher Darstellung wie Fig. 1, jedoch teilweise im Schnitt, eine Variante von Fig. 1 wieder.

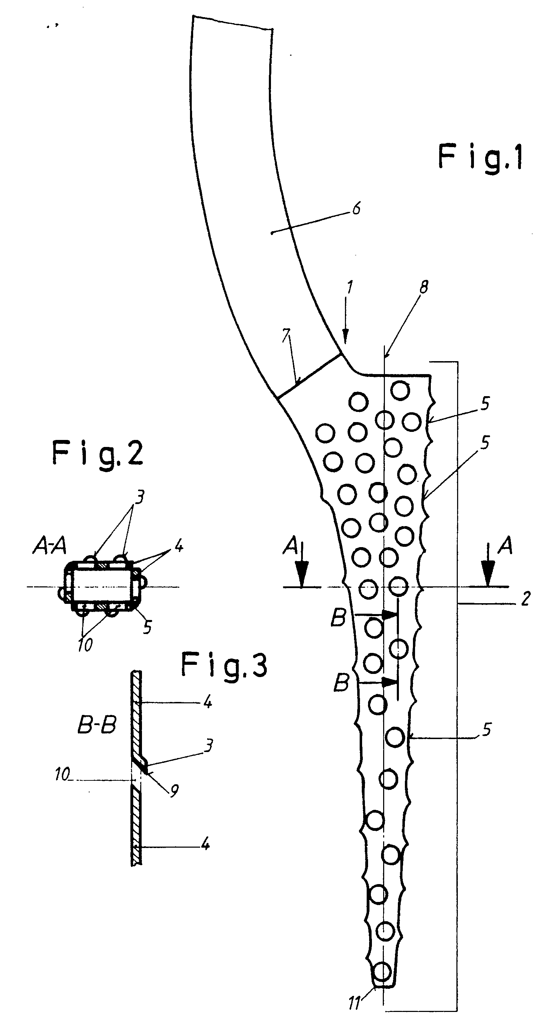

Das in Form und Abmessungen an den Schaft einer Femurkopfprothese angepasste Instrument 1 (Fig. 1) ist auf seiner Oberfläche im Arbeitsbereich 2 mit einer Vielzahl einzelner Zähne 3 versehen. Wie Fig. 2 zeigt, besteht der Arbeitsbereich 2 aus einem Hohlkörper, der aus fabrikatorischen Gründen aus einzelnen, im wesentlichen ebenen Blechen 4 zusammengesetzt ist, die durch einzelne, über die Länge verteilte Schweiss-Stellen 5 - entsprechend dem zugehörigen, nicht dargestellten Schaft - zu einem im wesentlichen rechteckigen Querschnitt zusammengefügt sind. Im Bereich, in dem bei einer Femurkopfprothese der Schaft in den Prothesenhals übergeht, ist an dem Hohlkörper ein massiver Oberteil 6, beispielsweise mit Hilfe einer Schweissnaht 7, befestigt; der Oberteil 6 wird in einem Bogen zu einem nicht dargestellten Amboss für ein Einschlaginstrument. Amboss und Oberteil sind dabei so ausgebildet, dass die Kraftwirkung der Schläge in Richtung der Einschlag- oder Längsachse 8 erfolgt.

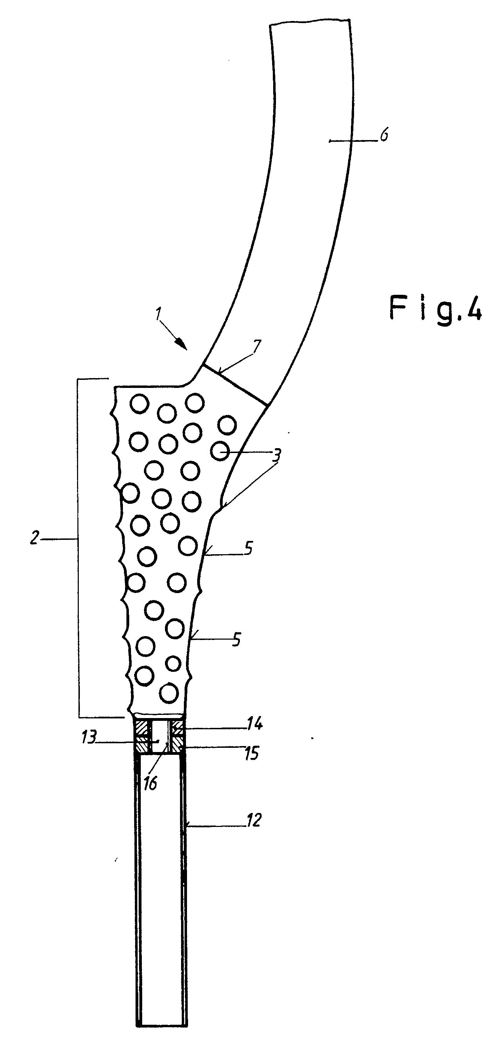

Die Zähne 3 enden in einer scharfen Schneidkante 9 (Fig. 3); darüberhinaus ist ihnen jeweils direkt ein Durchbruch 10 durch die Wand 4 des Hohlkörpers zugeordnet. Weiterhin kann das distale Ende des Hohlkörpers zusätzlich als Schneidkante 11 ausgebildet sein.

Bei dem Instrument nach Fig. 4 ist die Schaftlänge in einen proximalen Teil 2 und einen distalen Teil 12 unterteilt. In diesem Beispiel ist dabei der proximale Teil als mit Zähnen 3 versehener Arbeits- oder Schälbereich ausgeführt, während der distale Teil 12 ein zahnfreier glatter Führungskörper ist. Eine solche Ausbildung des Instruments wird beispielsweise bei Schäften bevorzugt, die im proximalen Bereich verankert werden und bei denen der distale Teil im Querschnitt kreisförmig ist und lediglich eine annähernd von Kraftübertragungen freie Gleitführung bildet.

Selbstverständlich ist es auch möglich, den Teil 12 ebenfalls als mit Zähnen 3 bestückten Schälbereich auszubilden.

Die Verbindung der Teile 2 und 12 kann beispielsweise über einen Gewindezapfen 13 erfolgen, der in einem scheibenförmigen Abschluss 14 des einen Teils 2 oder 12 befestigt ist. Das zugehörige Muttergewinde 16 trägt dann eine weitere Abschlussplatte 15 im anderen Teil 12 oder 2.

## Patentansprüche

1. Raspelartiges Räuminstrument (1) zum Vorbereiten eines Röhrenknochens für den Einsatz eines Implantatschaftes, welches Instrument über seine Arbeitslänge (2) in Umfangsrichtung scharfe Kanten hat, die beim Einschlagen in Richtung seiner Achse Knochenmaterial abschälen, wobei der Arbeitsbereich (2) des Instrumentes (1) als die Form des zu implantierenden Schaftes mindestens annähernd abbildender Hohlkörper ausgeführt ist, wobei ferner unter den Schneidkanten durch die Hohlkörperwand (4) in das Innere des Hohlkörpers führende Durchbrüche (10) vorgesehen sind, dadurch gekennzeichnet, dass die Schneidkanten zu einzelnen, in Umfangsrichtung verteilten Zähnen (3) aufgelöst sind, und dass ferner jedem Zahn (3) direkt ein Durchbruch (10) durch die Hohlkörperwand (4) zugeordnet ist.

2. Räuminstrument nach Anspruch 1, dadurch gekennzeichnet, dass der Hohlkörper aus einzelnen, längs Stosskanten mindestens stellenweise miteinander verschweissten Blechen (4) aufgebaut ist.

3. Räuminstrument nach Anspruch 1 oder 2, dadurch gekennzeichnet, dass es in Achsrichtung aus zwei Teilen (2, 12) zusammengesetzt ist.

4. Räuminstrument nach Anspruch 3, dadurch gekennzeichnet, dass der distale Teil (12) als zahnfreier Führungskörper ausgebildet ist.

## Claims

1. A rasp-like broaching instrument (1) for preparing a tubular bone for the insertion of an implant stem, the instrument having over its operative length (2) peripherally extending sharp edges which, when the instrument is knocked in axially, peel off bone material, the operative zone (2) of the instrument (1) being a hollow member which reproduces at least substantially the shape of the stem to be implanted, there being present below the cutting edges apertures (10) which extend through the wall (4) of the hollow member into the interior thereof, characterised in that the cutting edges are subdivided into discrete peripherally distributed teeth (3) and an aperture (10) through the wall (4) is directly associated with each tooth (3).

2. An instrument according to claim 1, characterised in that the hollow member is devised from individual metal members (4) which are welded together at least in places along abutting edges.

3. An instrument according to claim 1 or 2, characterised in that it is a combination in the axial direction of two parts (2, 12).

4. An instrument according to claim 3, characterised in that the distal part (12) is an untoothed guide member.

## Revendications

1. Instrument d'excavation (1) en forme de râpe destiné à la préparation d'un os long pour l'insertion d'une tige d'implantation d'une prothèse, ledit instrument comportant sur sa longueur de travail (2) des arêtes vives orientées dans la direction de la circonférence et qui détachent de la matière osseuse lorsque l'instrument est enfoncé dans la direction de son axe, la plage de travail (2) de l'instrument (1) étant conformée en corps creux reproduisant au moins approximativement la forme de la tige devant être implantée et, par ailleurs, des trous (10) traversant la paroi (4) du corps creux et débouchant à l'intérieur de ce dernier étant prévus sous les arêtes de coupe, caractérisé en ce que les arêtes de coupe sont réduites à de simples dents réparties dans la direction de la circonférence et en ce que, par ailleurs, chaque dent (3) est associée directement à un trou (10) traversant la paroi (4) du corps creux.

2. Instrument d'excavation selon la revendication 1, caractérisé en ce que le corps creux se compose de tôles individuelles (4) soudées au moins par points les unes aux autres le long de leurs bords d'assemblage.

3. Instrument d'excavation selon la revendication 1 ou 2, caractérisé en ce qu'il est formé d'un assemblage de deux parties (2, 12) dans la direction de l'axe.

4. Instrument d'excavation selon la revendication 3, caractérisé en ce que la partie distale (12) est conformée en corps de guidage ne com-

portant pas de dents.

Fig.1

Fig.2

Fig.3

Fig.4